# DEMANDE DE BREVET EUROPEEN

(11) **EP 0 698 391 A1**
(43) Date de publication de la demande: **28.02.1996**
(21) Numéro de dépôt: 95401738.0
(22) Date de dépôt: 24.07.1995
(51) Int. Cl.: A61K 31/445

(54) **Utilisation de l'ifenprodil et de ses diastéréoisomères pour la préparation de médicaments utiles dans le traitement des neuropathies périphériques et des maladies neurodégénératives centrales**

(30) Priorité: 29.07.1994 FR 9409410
(71) Demandeur: SYNTHELABO, F-92350 Le Plessis Robinson (FR)
(72) Inventeur: Benavides, Jésus, F-92290 Chatenay Malabry (FR); Ferzaz, Badia, F-92160 Antony (FR); George, Pascal, F-78730 Saint Arnould en Yvelines (FR); Scatton, Bernard, F-91120 Villebon sur Yvette (FR)
(74) Mandataire: Thouret-Lemaitre, Elisabeth

(57) **Abrégé**

Utilisation de l'ifenprodil et de ses diastéréoisomères pour la préparation de médicaments utiles dans le traitement des neuropathies périphériques et des maladies neurodégénératives centrales.

## Description

La présente invention a pour objet l'utilisation de l'ifenprodil et de ses diastéréoisomères, sous forme de base libre ou de sel d'addition à un acide pharmaceutiquement acceptable, pour la préparation de médicaments utiles dans le traitement des neuropathies périphériques et des maladies neurodégénératives centrales.

L'ifenprodil ou 1-(4-hydroxyphényl)-2-(4-phénylméthyl-1-pipéridyl)propanol, de formule (I) 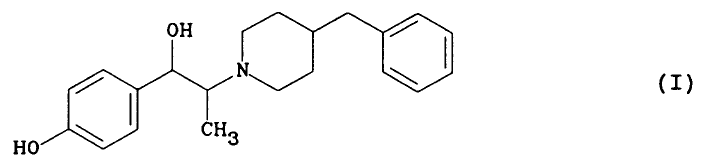
est un composé connu dont la préparation sous la forme érythro racémique, et les propriétés vasodilatatrices ont été décrites dans le brevet FR 5733 M.

Ce composé a également une activité anti-ischémique cérébrale, décrite en particulier dans J. Pharmacol. Exp. Ther., 247, 1211 (1988) et dans Brain Research, 522, 290 (1990).

La préparation des énantiomères de la forme érythro a été décrite dans le brevet FR 83 08290.
La préparation de la forme thréo racémique et de ses énantiomères a été décrite dans J. Med. Chem. **34**, 3085-3090, (1991).

La présente invention comprend l'utilisation des diastéréoisomères de l'ifenprodil, purs ou sous forme de mélange, y compris de mélange racémique.

Ces composés ont été soumis à de nouveaux essais pharmacologiques qui ont mis en évidence leurs propriétés neurotrophes.

En particulier, l'effet de l'ifenprodil sur la régénération du nerf sciatique in vivo a été étudié.
Cet effet a été évalué après lésion par congélation locale du nerf sciatique chez le rat.

La lésion par congélation détruit les fibres du nerf sciatique qui subissent une dégénérescence wallérienne au site de la lésion et dans tout le tronçon distal. Ce type de lésion conserve les gaines nerveuses et permet une régénération nerveuse dans des conditions reproductibles. Le processus de régénération commence à partir du côté proximal dans les heures qui suivent la lésion. La vitesse de régénération des fibres sensitives est mesurée par un test de pincement (pinch-test), 8 jours après la lésion.

Les animaux sont des rats adultes mâles de souche Sprague Dawley (Iffa Credo) pesant 250 g environ. Après anesthésie des animaux par du pentobarbital sodique (60 mg/kg), la peau de la cuisse est désinfectée à l'alcool et incisée au niveau de la jonction des biceps fémoraux. Le nerf sciatique est mis à nu après avoir écarté les muscles Lateralis et le Biceps Femoris. Le point de la lésion est repéré par une microsuture (Ethilon noir 10-0) sur le périnèvre au dessus de la trifurcation du nerf sciatique. La lésion du nerf sciatique est effectuée sur 1 mm par 6 cycles de congélation-décongélation à l'aide d'une cryode en cuivre pré-refroidie dans de l'azote liquide. La plaie est ensuite refermée et traitée par un antibiotique (Exoseptoplix®). Les animaux sont mis en cages individuelles et surveillés quotidiennement.

Après l'opération, les animaux sont répartis en 2 lots de 6 individus :
- animaux lésés qui reçoivent une injection i.p. de tween 80 à 0.1%, 10 minutes, 2 heures et 4 heures après la lésion, et deux injections par jour du deuxième au huitième jour.
- animaux lésés traités qui reçoivent une injection i.p. de tartrate (1:2) d'ifenprodil (3 mg/kg) dans du tween 80 à 0.1%, 10 minutes, 2 heures et 4 heures après la lésion, et deux injections par jour à partir du deuxième jour.

Huit jours après l'opération, les animaux sont légèrement anesthésiés, et le nerf sciatique est remis à nu pour effectuer le pinch-test. Ce test consiste à pincer légèrement le nerf à l'aide de forceps en commençant par la région la plus distale du nerf et en remontant tous les 0,5 mm. Une réponse reflexe (contraction des muscles de la région postérieure de l'animal) est observée là où le front des fibres sensitives régénérées est présent. Ce point est marqué par une microsuture. Le nerf est ensuite prélevé, la distance entre le site de la lésion et la microsuture distale est mesurée sur un papier millimétré sous microscope opératoire. Après prélèvement du nerf, les animaux sont sacrifiés par une overdose de pentobarbital.

Chez les témoins lésés, le pinch-test effectué 8 jours après la lésion a montré une réaction des animaux à 26 mm en moyenne ; cette longueur correspond à la distance parcourue par les fibres sensitives régénérées en 8 jours. Chez les animaux traités au tartrate (1:2) d'ifenprodil, l'injection deux fois par jour de 3 mg/kg augmente la distance parcourue par les fibres sensitives de 14,7 %.

Les résultats des essais effectués suggèrent que l'ifenprodil et ses diastéréoisomères possèdent des activités neurotrophes.
Ces composés peuvent donc être utilisés pour la préparation de médicaments utiles dans le traitement des neuropathies périphériques de type traumatique, ischémique, métabolique, infectieux, alcoolique, iatrogénique ou génétique, et dans le traitement des maladies affectant les motoneurones, comme la sclérose latérale amyotrophique et les amyotrophies spinales.

Ils peuvent aussi être utilisés pour la préparation de médicaments utiles dans le traitement de la sénilité cérébrale, de la démence consécutive aux infarctus multiples, de la démence vasculaire, de la sclérose en plaques, dans le traitement de l'atrophie olivo-ponto-cérébelleuse et d'autres maladies neurodégénératives, par exemple la maladie d'Alzheimer, la maladie de Pick ou la chorée de Huntington.

L'ifenprodil et ses diastéréoisomères peuvent être utilisés seuls ou en association avec d'autres substances thérapeutiques, par exemple avec un agent anticancéreux, en vue de réduire les effets secondaires (neuropathies et autres) de ce dernier.

A cet effet, ils peuvent être présentés sous toutes formes pharmaceutiques adaptées à l'administration entérale, parentérale ou locale, en association avec des excipients appropriés, par exemple sous forme de comprimés, dragées, gélules, capsules, suppositoires, patches, solutions ou suspensions buvables ou injectables, dosés pour permettre une administration journalière de 1 à 1000 mg de principe actif.

## Revendications

1. Utilisation de l'ifenprodil et de ses diastéréoisomères, sous forme de base libre ou de sel d'addition à un acide pharmaceutiquement acceptable, pour la préparation de médicaments utiles dans le traitement des neuropathies périphériques et des maladies neurodégénératives centrales.
